(19) **Europäisches Patentamt**
**European Patent Office**
**Office européen des brevets**

(11) **EP 2 442 106 B1**

(12) **EUROPEAN PATENT SPECIFICATION**

(45) Date of publication and mention
of the grant of the patent:
**19.08.2015 Bulletin 2015/34**

(21) Application number: **10785955.5**

(22) Date of filing: **09.06.2010**

(51) Int Cl.:
*G01N 33/53* (2006.01)     *G01N 33/493* (2006.01)
*G01N 33/70* (2006.01)     *G01N 33/68* (2006.01)

(86) International application number:
**PCT/JP2010/003837**

(87) International publication number:
**WO 2010/143423 (16.12.2010 Gazette 2010/50)**

(54) **METHOD FOR TEST ON DIABETIC NEPHROPATHY**

TESTVERFAHREN AUF DIABETES-NEPHROPATHIE

MÉTHODE DE DÉTECTION D'UNE NÉPHROPATHIE DIABÉTIQUE

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **10.06.2009 JP 2009139188**

(43) Date of publication of application:
**18.04.2012 Bulletin 2012/16**

(73) Proprietors:
• **Hara, Masanori**
**Niigata 950-2041 (JP)**
• **Niigata University**
**Niigata 950-2181 (JP)**
• **Juntendo Educational Foundation**
**Tokyo 113-8421 (JP)**
• **DENKA SEIKEN CO., LTD.**
**Tokyo 103-0025 (JP)**

(72) Inventors:
• **HARA, Masanori**
**Niigata-shi**
**Niigata 950-2041 (JP)**
• **SAITO, Akihiko**
**Niigata-shi**
**Niigata 951-8510 (JP)**
• **TOMINO, Yasuhiko**
**Tokyo 113-8421 (JP)**
• **ASANUMA, Katsuhiko**
**Tokyo 113-8421 (JP)**
• **KUROSAWA, Hiroyuki**
**Gosen-shi**
**Niigata 959-1695 (JP)**

• **OGASAWARA, Shinya**
**Gosen-shi**
**Niigata 959-1695 (JP)**
• **HIRAYAMA, Yoshiaki**
**Gosen-shi**
**Niigata 959-1695 (JP)**

(74) Representative: **Zwicker, Jörk et al**
**ZSP Patentanwälte**
**Partnerschaftsgesellschaft**
**Radlkoferstrasse 2**
**81373 München (DE)**

(56) References cited:
**WO-A1-02/37099     WO-A1-2007/102787
JP-A- 6 011 507     JP-A- 2008 122 410**

• **TSUKASA NAKAMURA ET AL: "Pioglitazone
reduces urinary podocyte excretion in type 2
diabetes patients with microalbuminuria",
METABOLISM, vol. 50, no. 10, 1 October 2001
(2001-10-01), pages 1193-1196, XP055048587,
Elsevier Inc New York Ny USA ISSN: 0026-0495,
DOI: 10.1053/meta.2001.26703**
• **HARA M ET AL: "Urinary podocalyxin is an early
marker for podocyte injury in patients with
diabetes: establishment of a highly sensitive
ELISA to detect urinary podocalyxin",
DIABETOLOGIA ; CLINICAL AND
EXPERIMENTAL DIABETES AND METABOLISM,
SPRINGER, BERLIN, DE, vol. 55, no. 11, 2 August
2012 (2012-08-02), pages 2913-2919,
XP035121284, Heidelberg & New York ISSN:
1432-0428, DOI: 10.1007/S00125-012-2661-7**

• MASANORI HARA: 'Jin Shikkan no Shinryo ni Yakudatsu Atarashii Kensa Nyochu Podocyte Kensa, Nyochu Podocalyxin Teiryo' KIDNEY AND DIALYSIS vol. 65, no. 4, 25 October 2008, pages 488 - 491, XP008149076

• NAKAMURA T. ET AL.: 'Urinary excretion of podocytes in patients with diabetic nephropathy' NEPHROL.DIAL.TRANSPLANT. vol. 15, no. 9, 2000, pages 1379 - 1383, XP009065526

• SHIGERU SEKINE ET AL.: 'Jin Shogai no Atarashii Marker ni Tsuite -Nyochu Podocyte, Podocalyxin to Rinsho Byorigakuteki Igi-' THER.RES. vol. 29, no. 11, 2008, pages 1900 - 1904, XP008148139

• MASANORI HARA: 'Nyo Tanpaku Nyochu Bio Marker (1)Podocyte Kensa, Nyochu Podocalyxin Teiryo Kensa' JINZO vol. 32, no. 3, 25 March 2010, pages 196 - 200, XP008150661

**Description**

Technical Field

The present invention relates to a test method for diabetic nephropathy, including detecting urinary podocalyxin, and a test reagent for diabetic nephropathy for use in the test method, including an anti-podocalyxin antibody.

Background Art

Diabetic nephropathy is one of the three major complications of diabetes, and has been the leading cause for initiation of hemodialysis in recent years. Diabetic nephropathy develops when, ten or more years after the onset of diabetes, the diabetes is poorly controlled and a hyperglycemic condition lasts for a long period of time. An increase in glomerular filtration rate, an increase in intraglomerular pressure (glomerular hypertension), and an increase in permeability of a plasma protein from the glomerular capillaries lead to the occurrence of albuminuria. The further progression of the condition loosens the network of the glomerular basement membrane, and thus even a protein having a larger size starts tobe excreted, resulting inproteinuria. Severe proteinuria leads to hypoproteinemia, which causes edema synergistically with a decrease in glomerular filtration function. The still further progression of the condition impairs the excretion of body wastes, water, and salts, resulting in a renal failure condition, which finally requires hemodialysis or renal transplantation.

The proportion and number of patients with diabetic nephropathy as the primary disease of end-stage renal failure have been increasing year by year, and the diagnosis and treatment of diabetic nephropathy are large problems. A dialysis patient with diabetic nephropathy often has a poor prognosis, and a dialysis therapy does not necessarily make a large contribution to the life prognosis and QOL improvement of the patient at present. Further, the clinical diagnosis of diabetic nephropathy is performed based on findings of persistent proteinuria, renal dysfunction, hypertension, and the like complicated with diabetes because of a difficulty of renal biopsy. However, once persistent proteinuria occurs, it is difficult to treat the condition, resulting in end-stage renal failure in many cases after a lapse of five to six years. Therefore, from the standpoint of clinical medicine, there is a strong demand for the diagnosis and treatment of renal lesions at an early stage before a urine protein is judged as positive in a conventional dipstick test.

As a technique for diagnosing a patient with diabetic nephropathy at an early stage at which a urine protein is judged as negative in the dipstick test, there is performed urinary albumin measurement using radioimmunoassay, enzyme-linked immunoassay, latex agglutination immunoturbidimetry, or immunoprecipitation. As an indicator for such technique, a urinary albumin excretion rate albumin/creatinine ("ALB/Cre") is used. The urinary albumin excretion rate (mg/g of Cre) can be determined with $100 \times$ urinary albumin concentration (mg/L) /urinary creatinine concentration (mg/dL). A case where the urinary albumin excretion rate "ALB/Cre" is less than 30 mg/g of Cre corresponds to Stage I (pre-nephropathy), a case where the urinary albumin excretion rate is 30 to less than 300 mg/g of Cre corresponds to Stage II (incipient nephropathy), and a case where the urinary albumin excretion rate is 300 mg/g of Cre or more corresponds to Stage III (overt nephropathy). A case where the urinary albumin excretion rate is less than 30 mg/g of Cre is assessed to correspond to a stage at which nephropathy has not developed. When a subject to be tested (hereinafter, sometimes referred to as "subject") shows microalbumin, the subj ect is diagnosed to have diabetic nephropathy, which corresponds to Stage II, a stage at which pathologically mild to moderate diffuse lesions have already existed and the presence of nodular lesions is also known. Thus, real initial nephropathy cannot necessarily be judged by the diagnosis of urinary albumin, and there is a demand for an indicator capable of diagnosing diabetic nephropathy at an early stage.

There is disclosed a method involving using, as a diagnostic indicator, a von Willebrand factor cleaving enzyme in place of the urinary albumin concentration (Patent Literature 1). Specifically, regarding the amount or enzymatic activity of the von Willebrand factor cleaving enzyme, there is disclosed a measure for predicting the degree of progression of renal injury and improving a prognosis in a patient with diabetic nephropathy by analyzing the amount of vWF using an immunological technique.

There is disclosed a simple test measure for renal injury involving measuring urinary podocalyxin in association with renal diseases (Patent Literature 2). Podocalyxin is a sugar protein which is present in the surface of podocytes constructing the renal glomerulus and is responsible for a filtration function. The podocytes are located on the Bowman's space side in the glomerular basement membrane and play important roles in the mechanism of glomerular filtration. Thus, it is known that the grasping of the degree of injury in the podocytes has an extremely important meaning in understanding renal diseases (Non Patent Literature 1).

WO 2007/102787 A1 discloses a method of identifying an undifferentiated human embryonic stem cell in a sample by identifying those cells in the sample that express podocalyxin-like protein (PODXL) on their surface. Also disclosed is a kit comprising an anti-PODXL antibody (Patent literature 3)

Nakamura and coworkers stated that detection of podocytes in urine indicates severe injury to podocytes in the glomeruli in various renal diseases. They found that antidiabetic agent pioglitazone reduces urinary podocyte excretion in type 2 diabetes patients with microalbuminuria (Non Patent Literature 2). Hara and coworkers described that the process of glomerular sclerosis could be predicted by monitoring podocytes in urine. Podocytes in urine could be used as a treatment marker for diabetic nephropathy (Non Patent Literature 3)

Nakamura et al. examined whether podocytes are present in the urine sediments of adult diabetic patients with and without nephropathy (Non Patent Literature 4). They speculate that podocytes in the urine may be a useful marker of disease activity in diabetic nephropathy.

Sekine et al, describe a formula in which the podocalyxin amount is divided by the creatinine amount. The value obtained is used for the assessment of the podocalyxin concentration in urine (Non Patent Literature 5).

Citation List

Patent Literature

[PTL 1] JP 2008-216137 A
[PTL 2] WO 2002/037099 A1
[PTL 3] WO 2007/102787 A1

Non Patent Literature

[NPL 1] Hara et al., Nephron69: 397-403 (1995)
[NPL 2] Nakamura et al., Metabolism 50(10): 1193-1196 (2001)
[NPL 3] Hara et al., Kidney and Dialysis, 65(4):488-491 (2008)
[NPL 4] Nakamura et al., Nephrol. Dial. Transplant., 15 (9) : 1379-1383 (2000)
[NPL 5] Sekine et al., Ther. Res., 29(11): 1900-1904 (2008)

Summary of Invention

Technical Problem

An object of the present invention is to provide a test method for the detection of diabetic nephropathy at an early stage as compared to a conventional method.

Solution to Problem

In order to solve the above-mentioned problem, the inventors of the present invention have focused on the fact that, when a subject has a urinary podocalyxin excretion rate "PCX/Cre" higher than a value therefor in a healthy subject, the subject is suspected of having diabetic nephropathy, and have found that diabetic nephropathy can be tested by detecting urinary podocalyxin. Thus, the present invention has been completed.

That is, the present invention includes the following items.

1. A test method for diagnosing diabetic nephropathy, comprising:

detecting solubilized urinary podocalyxin,
wherein the urinary podocalyxin value is corrected with a urinary creatinine amount or concentration by dividing the urinary podocalyxin amount or concentration by the urinary creatinine amount or concentration, respectively; and
assessing a subject to be tested who has a urinary podocalyxin value higher than a reference value to have diabetic nephropathy, wherein the reference value is a urinary podocalyxin value in a healthy subject.

2. A test method according to the item 1, wherein an early stage of the diabetic nephropathy is detected, wherein said early stage is characterized by a urinary albumin excretion rate "ALB/Cre" of less than 30 mg/g of creatinine.

3. A test method according to the item 1 or 2, wherein the reference value comprises an upper limit value of a 95% confidence interval of a urinary podocalyxin value in a healthy subject.

4. A test method for diabetic nephropathy according to the item 1 or 2, further comprising assessing progression of the diabetic nephropathy and/or classifying the diabetic nephropathy into stage I (pre-nephropathy), Stage II (incipient nephropathy), or Stage III (overt nephropathy), wherein a urinary albumin excretion rate "ALB/Cre" of less than 30 mg/g of Cre corresponds to Stage I (pre-nephropathy), a urinary albumin excretion rate from 30 to less than 300 mg/g of Cre corresponds to Stage II (incipient nephropathy), and a urinary albumin excretion rate of 300 mg/g of Cre or more corresponds to Stage III (overt nephropathy).

5. A test method according to any one of the items 1 to 4, wherein the detecting of the urinary podocalyxin is carried out by an immunological technique.

Advantageous Effects of Invention

The test method of the present invention can be performed by measuring urinary podocalyxin, thereby detecting diabetic nephropathy at an early stage. The test method of the present invention allows even a patient classified into Stage I (pre-nephropathy) with a urinary albumin excretion rate "ALB/Cre" to be reclassified into a patient at a high risk of nephropathy. The detection of diabetic nephropathy at an early stage by the test method of the present invention contributes to the prevention of nephropathy progression as well.

Further, the urinary podocalyxin excretion rate "PCX/Cre" tends to increase along with the progression of diabetic nephropathy, and the condition of diabetic nephropathy can be grasped by the test method of the present invention. The urinary podocalyxin excretion rate reflects glomerular hyperfunction and the subsequent progressive injury, and hence can be used for effectively checking active glomerular injury. It is conceivable that the test method of the present invention can simply monitor the condition of diabetic nephropathy and rapidly determine a therapeutic strategy or the like.

Brief Description of Drawings

[FIG. 1] FIG. 1 is a graph illustrating urinary podocalyxin excretion rates "PCX/Cre" of patients with diabetes and diabetic nephropathy classified in accordance with the staging of diabetic nephropathy (Example 2).

[FIG. 2] FIG. 2 is a graph illustrating urinary podocalyxin excretion rates "PCX/Cre" of patients with diabetes and diabetic nephropathy except those with eGFR<60 classified in accordance with the staging of diabetic nephropathy (Example 2).

Description of Embodiments

The present invention includes testing diabetic nephropathy by detecting urinary podocalyxin of a subject. In this description, urine, which is used as a specimen, may be obtained from any subj ect, but is preferably obtained from a patient suffering from diabetes. The diagnosis of diabetes has only to be performed by a conventionally known measure. No particular limitation is imposed on a collection method for urine, but it is preferred to use early morning urine or casual urine. Further, the amount of urine necessary for the test method of the present invention is about 10 to 200 μL. The test method of the present invention may be performed concurrently with a general urine test to be conventionally performed in a medical examination or the like, or may be performed by separately collecting urine from a subject suspected of having diabetes or a patient judged to have diabetes. Further, the test method of the present invention may be performed for the purpose of monitoring the development or progression of diabetic nephropathy in a patient with diabetes.

Urine as a specimen may be treated by adding and mixing a treatment liquid into the urine. The treatment liquid may be any as long as the pH adjustment of the urine, the masking of a urine sediment, and the solubilization of podocalyxin are possible, but is preferably exemplified by a solution obtained by adding a chelating agent, a surfactant, and the like to a buffer. The buffer and the chelating agent may be any known buffer and chelating agent, and it is preferred to use a nonionic surfactant as the surfactant. The treatment liquid is exemplified by a solution including 0.2 M EDTA and 2% (Vol./Vol.) Triton X-100 in 2 M TES-NaOH (pH 7.0). A urine sample solution can be obtained by adding and mixing 10 μL of such treatment liquid into 90 μL of a urine specimen.

Various methods may be employed as a detection method for podocalyxin in the urine sample solution. An example of the detection method for the urinary podocalyxin is an immunological technique. The immunological technique may be performed, for example, by an immunostaining method (including a fluorescent antibody method, an enzymatic antibody method, a heavy metal-labeled antibody method, and a radioisotope-labeled antibody method), a combination of separation based on an electrophoresis method and a detection method with fluorescence, an enzyme, a radioisotope, or the like (including a western blot method and a fluorescent two-dimensional electrophoresis method), enzyme-linked immunosorbent assay (ELISA), a dot blotting method, latex agglutination-turbidimetric immunoassay (LA), or immunochromatography. Of those, it is preferred to employ an ELISA method or an LA method. It is preferred to employ a sandwich method in the ELISA method from the viewpoint of quantitative property. In the sandwich method, a urine sample solution is added to an anti-podocalyxin antibody-coated microtiter plate to cause an antigen-antibody reaction, an enzyme-labeled anti-podocalyxin antibody is further added to cause an antigen-antibody reaction, the plate is washed and then subjected to a reaction with an enzyme substrate and color development, the absorbance is measured to detect urinary podocalyxin, and the measured value can be used to calculate a urinary podocalyxin concentration.

The anti-podocalyxin antibody for use in the immunological technique has only to be an antibody capable of detecting podocalyxin. The anti-podocalyxin antibody for use in the present invention is not particularly limited, and may be a known antibody or an antibody to be developed in the future. Examples thereof include monoclonal and polyclonal antibodies, a labeled antibody, a chimeric antibody, a humanized antibody, and binding active fragments thereof.

A value for urinary podocalyxin for use in the present invention may be a urinary podocalyxin concentration, but is desirably a urinary podocalyxin concentration corrected with a value for a urinary component to be stably excreted in urine (urinary component value). The urinary component is particularly preferably urinary creatinine. It is considered that urinary creatinine is substantially constant irrespective of a disease in one individual because the production of creatinine depends on the amount of a muscle. In a test for a urinary excretion substance, in order to eliminate an error in urinary amount, a technique involving correcting the amount of a urinary excretion substance of interest with an amount per g of creatinine is generally employed. This allows the comparison of urinary excretion substances per unit gram of creatinine. A corrected value obtained by correcting a urinary podocalyxin concentration with a urinary creatinine concentration is referred to as urinary podocalyxin excretion rate (PCX/Cre), and the urinary podocalyxin excretion rate can be calculated with the following equation.

$$\text{<Equation> PCX/Cre: urinary podocalyxin excretion rate}$$
$$(\mu g/g) = 100 \times \text{urinary podocalyxin concentration (ng/mL)/urinary}$$
$$\text{creatinine concentration (mg/dL)}$$

When a subject has a urinary podocalyxin value, preferably a urinary podocalyxin excretion rate "PCX/Cre", which is obtained by the test method of the present invention, higher than a reference value, the subject can be assessed to have diabetic nephropathy. The reference value may be appropriately set, but a urinary podocalyxin value, preferably a urinary podocalyxin excretion rate "PCX/Cre" in a healthy subject may be used. The healthy subject may include a patient with diabetes who has not developed diabetic nephropathy as well as a subject except a patient with diabetes. However, it is preferred that the healthy subject be desirably a subject except a patient with diabetes, more desirably a subject negative for an additional renal function marker except a patient with diabetes. Examples of the additional renal function marker include an estimated glomerular filtration rate (eGFR) and a urine protein. It is more preferred that urinary podocalyxin values, preferably urinary podocalyxin excretion rates "PCX/Cre" be determined from a plurality of healthy subjects and the upper limit value of the 95% confidence interval of the urinary podocalyxin excretion rates be used as a reference value.

The 95% confidence interval may be determined by a known technique. When urinary podocalyxin values in healthy subjects follow a normal distribution, the 95% confidence interval thereof can be determined with the following equation.

$$95\% \text{ confidence interval} = \text{average of urinary podocalyxin values}$$
$$\text{in healthy subjects} \pm t \times \text{standard deviation of urinary podocalyxin}$$
$$\text{values in healthy subjects}$$

It should be noted that t represents a degree of freedom and varies depending on the number of specimens of healthy subjects, and hence has only to be selected based on a t-distribution table. In general, t represents 1.96 in the case of the 95% confidence interval.

Meanwhile, when urinary podocalyxin values in healthy subjects do not follow a normal distribution, a range that accounts for 95% including the median is defined as a reference range, and the upper limit value of the reference range is defined as a reference value for the urinary podocalyxin values.

In the present invention, the reference value is 50 $\mu$g/g to 300 $\mu$g/g, preferably 75 $\mu$g/g to 200 $\mu$g/g, more preferably 100 $\mu$g/g to 180 $\mu$g/g.

The stages of diabetic nephropathy are generally classified into Stage I (pre-nephropathy), Stage II (incipient nephropathy), and Stage III (overt nephropathy). In Stage I (pre-nephropathy), a urinary albumin value is normal, and as histopathological features, no or mild diffuse lesions exist. In Stage II (incipient nephropathy), it is known that microalbuminuria occurs, and as histopathological features, mild to moderate diffuse lesions exist and nodular lesions exist in some cases. In Stage III (overt nephropathy), it is known that persistent proteinuria occurs, and as histopathological features, moderate to severe diffuse lesions exist and nodular lesions exist in many cases.

The test method of the present invention can assess diabetic nephropathy at Stage I as well as Stage II and Stage III in the above-mentioned staging. In the case of conventional staging with urinary albumin measurement, Stage I is assessed as a stage at which nephropathy has not developed. In contrast, the test method of the present invention can assess Stage I, which allows diabetic nephropathy to be diagnosed at an early stage.

Further, the urinary podocalyxin excretion rate "PCX/Cre" reflects glomerular hyperfunction under progression and the subsequent progressive injury. Therefore, an increase in the urinary podocalyxin excretion rate can be confirmed to assess and/or predict the progression of the condition of diabetic nephropathy. When the progression of the condition of diabetic nephropathy is assessed and/or predicted, it is preferred to measure a urinary podocalyxin value in a subject with time, and an increase in the urinary podocalyxin value is assessed as the progression of the condition of diabetic nephropathy. Further, the staging of diabetic nephropathy may also be performed based on the urinary podocalyxin excretion rate.

A method involving assessing and/or predicting the progression of the condition of diabetic nephropathy or performing the staging of diabetic nephropathy with use of glomerular hyperfunction under progression is preferably applied to a subject who has active glomeruli and is free of advanced glomerulosclerosis. Such subject is preferably a subject negative for an additional renal function marker, and is exemplified by a subject with eGFR of 60 or more.

Also disclosed herein are a test reagent for diabetic nephropathy for use in the test method, including an anti-podocalyxin antibody for detecting urinary podocalyxin, and a test reagent kit for diabetic nephropathy for use in the test method, including a reagent including an anti-podocalyxin antibody. The anti-podocalyxin antibody included in the test reagent or the test reagent kit may be labeled, for example, with an enzyme or the like. Further, the test reagent kit may include two or more kinds of anti-podocalyxin antibodies, and the antibodies are preferably antibodies capable of recognizing epitopes different from each other. In addition, the kit may include a reagent such as a treatment liquid or a chromogenic substrate, an instrument necessary for a test, and the like.

Examples

Hereinafter, the present invention is further specifically described by way of examples of the present invention. However, the present invention is by no means limited thereto, and various applications are possible without departing from the technical idea of the present invention.

(Example 1) Measurement of urinary podocalyxin concentration

A podocalyxin concentration was measured using two kinds of anti-human podocalyxin monoclonal antibodies. Those two kinds of antibodies recognize different two epitopes of human podocalyxin, respectively, and are an anti-human podocalyxin monoclonal antibody a (hereinafter, simply referred to as "antibody a") and an anti-human podocalyxin monoclonal antibody b (hereinafter, simply referred to as "antibody b"), respectively. In this example, an antibody a-coated microtiter plate (split type micro plate GF8 high: Nunc) and a horseradish peroxidase (hereinafter, abbreviated as "HRP")-labeled antibody b were used.

First, 90 $\mu$L of urine obtained from a subject were mixed with 10 $\mu$L of a solution of 2 M TES-NaOH, 0. 2 M EDTA, and 2% (Vol. /Vol.) Triton X-100, pH 7.0. 100 $\mu$L of a urine sample solution obtained by the mixing were added to wells of an antibody a-coated microtiter plate. The plate was left to stand still at 37°C for 1 hour, and the urine sample solution was then removed by decantation from the wells. Washing was performed by adding 3. 6 mM $Na_2HPO_4$, 1. 4 mM $KH_2PO_4$, 145 mMNaCl, and 0.05% (Vol./Vol.) Tween 20 (hereinafter, abbreviated as "PBS-T") to the wells of the microtiter plate at 200 $\mu$L/well and removing PBS-T by decantation. The washing step was performed a total of three times. After that, an HRP-labeled antibody b solution was added at 100 $\mu$L/well. The plate was left to stand still at 37°C for 1 hour, and the HRP-labeled antibody b solution was then removed by decantation. Washing was performed by adding PBS-T at 200 $\mu$L/well and removing PBS-T by decantation. The washing step was performed a total of three times. After that, a TMB One-Step Substrate System (Dako) was used as a substrate solution for an HRP enzymatic reaction and added at 100 $\mu$L/well, and the plate was left to stand still under a light-shielding condition at 25°C for 30 minutes. After that, a 313 mM $H_2SO_4$ solution was added at 100 $\mu$L/well as a reaction terminating solution, and each of the wells was measured for its absorbances at wavelengths of 450 nm and 630 nm using Multiskan Ascent and Ascent Software for Multiskan (Dainippon Pharmaceutical Co., Ltd.). Then, a value obtained by subtracting the absorbance at a wavelength of 630 nm from the absorbance at a wavelength of 450 nm was defined as a measured value. Native human podocalyxin extracted from the kidney was used as a standard for a calibration curve to derive a podocalyxin concentration in a specimen. Then, a urinary podocalyxin excretion rate, in which a urinary podocalyxin concentration was corrected with a urinary creatinine concentration, was calculated with the following equation.

<Equation> PCX/Cre: urinary podocalyxin excretion rate (μg/g)=100×urinary podocalyxin concentration (ng/mL)/urinary creatinine concentration (mg/dL)

(Example 2) Clinical significance of urinary podocalyxin excretion rate in diabetic nephropathy

The urinary podocalyxin excretion rates (creatinine-corrected values) of 71 patients with diabetes and diabetic nephropathy were determined by the method of Example 1 and classified in accordance with the staging of diabetic nephropathy. A group of healthy subjects is defined as Group A, a group of patients with diabetic nephropathy at Stage I (pre-nephropathy) is defined as Group B, a group of patients with diabetic nephropathy at Stage II (incipient nephropathy) is defined as Group C, and a group of patients with diabetic nephropathy at Stage III (overt nephropathy) is defined as Group D. This means that a specimen, in which the urinary podocalyxin excretion rate "PCX/Cre" determined by the method of Example 1 showed a higher value than a reference value, has diabetic nephropathy. The reference value is the upper limit value of the 95% confidence interval of urinary podocalyxin excretion rates "PCX/Cre" determined from urine collected from 66 healthy subjects (excluding a patient with diabetes in this example). The reference value is 161 μg/g.

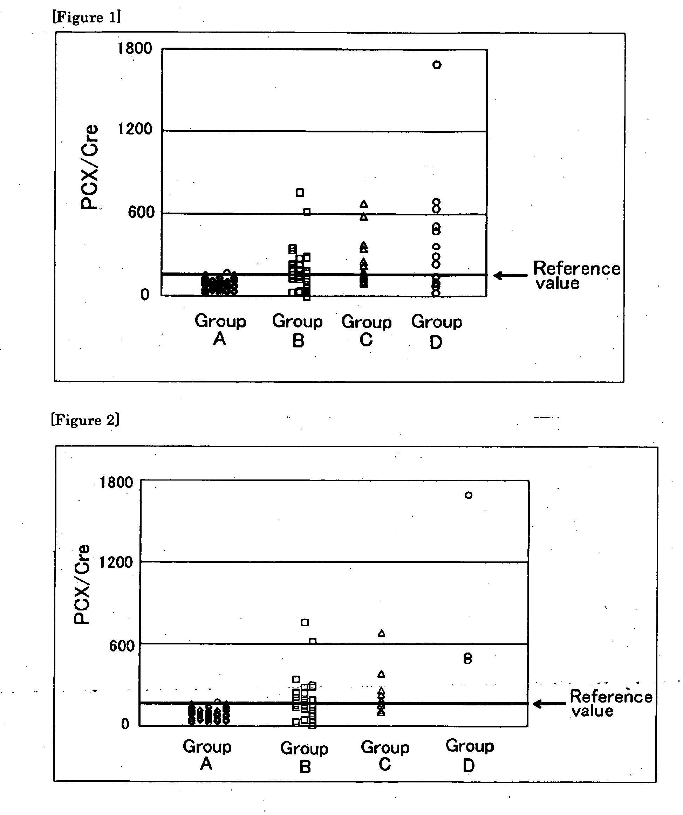

Table 1 and FIG. 1 show the urinary podocalyxin excretion rates "PCX/Cre" of patients with diabetes and diabetic nephropathy classified in accordance with the staging of diabetic nephropathy.

[Table 1]

|  | Healthy subject | Diabetic nephropathy | | |
|---|---|---|---|---|
|  |  | Stage I (pre-nephropathy ) | Stage II (incipient nephropathy) | Stage III (overt nephropathy) |
| Urinary podocalyxin excretion rates equal to or more than reference value (Number of cases) | 1/66 cases | 18/39 cases | 8/17 cases | 9/15 cases |
| Urinary podocalyxin excretion rates equal to or more than reference value (%) | 1.5% | 46.2% | 47.1% | 60.0% |

Table 1 and FIG. 1 revealed that the percentage of specimens having urinary podocalyxin excretion rates "PCX/Cre"equal to or more than the reference value became higher as the condition got worse. The table and figure also revealed that patients having urinary podocalyxin excretion rates "PCX/Cre" equal to or more than the reference value had been present at 46.2% at the normoalbuminuric stage (Stage I) before the occurrence of microalbuminuria. This means that podocalyxin has been excreted in urine before the occurrence of microalbuminuria. The results revealed that the detection of urinary podocalyxin allowed a patient classified into Stage I (pre-nephropathy) in accordance with the staging of diabetic nephropathy to be reclassified into a patient at a high risk of nephropathy.

Next, examination was made as to whether or not the urinary podocalyxin excretion rate "PCX/Cre" was able to be used for checking active glomerular injury. For that purpose, among the 71 patients with diabetes and diabetic nephropathy, 46 patients except patients with eGFR<60 estimated to have advanced glomerulosclerosis and to have a decreased gap space on the Bowman's space side capable of allowing urinary excretion were each subjected to sub-analysis for their urinary podocalyxin excretion rate "PCX/Cre."

Table 2 and FIG. 2 show the urinary podocalyxin excretion rates "PCX/Cre" of the patients with diabetes and diabetic nephropathy except those with eGFR<60 classified in accordance with the staging of diabetic nephropathy.

[Table 2]

| | Healthy subject | Diabetic nephropathy | | |
|---|---|---|---|---|
| | | Stage I (pre-nephropathy ) | Stage II (incipient nephropathy) | Stage III (overt nephropathy) |
| Urinary podocalyxin excretion rates equal to or more than reference value (number of cases) | 1/66 cases | 15/33 cases | 6/10 cases | 3/3 cases |
| Urinary podocalyxin excretion rates equal to or more than reference value (%) | 1.5% | 45.5% | 60.0% | 100.0% |

As seen from a comparison between the results of the 71 patients with diabetes and diabetic nephropathy in the staging of diabetic nephropathy and the results of the 46 patients except the patients with eGFR<60, the percentages of patients having urinary podocalyxin excretion rates "PCX/Cre" equal to or more than the reference value are equivalent in both the patients at Stage I, whereas much higher values are observed in the 46 patients except the patients with eGFR<60 at Stage II and Stage III (Table 1, Table 2, FIG. 1, and FIG. 2). In other words, the comparison revealed that the urinary podocalyxin excretion rate reflected glomerular hyperfunction under progression and the subsequent progressive injury. In other words, a test method suitable for checking active glomerular injury can be provided by the use of the urinary podocalyxin excretion rate "PCX/Cre."

Industrial Applicability

As described above, the urinary podocalyxin excretion rate "PCX/Cre" was found to reflect the development of diabetic nephropathy with high sensitivity at an early stage as compared to the urinary albumin excretion rate "ALB/Cre," which has been used as a diagnostic indicator for diabetic nephropathy. Further, the use of the urinary podocalyxin excretion rate "PCX/Cre" as a diagnostic marker for diabetic nephropathy can detect diabetic nephropathy at an early stage and accurately determine the degree of injury (progression of the condition). The test method of the present invention is useful because the method can prevent diabetic nephropathy from advancing in severity, reduce a medical treatment cost, and make a rapid decision on a therapeutic strategy or the like.

**Claims**

1. A in vitro test method for diagnosing diabetic nephropathy, comprising:

    detecting solubilized urinary podocalyxin, wherein the urinary podocalyxin value is corrected with a urinary creatinine amount or concentration by dividing the urinary podocalyxin amount or concentration by the urinary creatinine (Cre) amount or concentration, respectively; and
    assessing a subject to be tested who has a corrected urinary podocalyxin value higher than a reference value to have diabetic nephropathy, wherein the reference value is a urinary podocalyxin value in a healthy subject.

2. A test method according to claim 1, wherein an early stage of the diabetic nephropathy is detected, wherein said early stage is **characterized by** a urinary albumin (ALB) excretion rate "ALB/Cre" of less than 30 mg/g of creatinine.

3. A test method according to claim 1 or 2, wherein the reference value comprises an upper limit value of a 95% confidence interval of a urinary podocalyxin value in a healthy subject.

4. A test method for diabetic nephropathy according to claim 1 or 2, further comprising assessing progression of the diabetic nephropathy and/or classifying the diabetic nephropathy into stage I (pre-nephropathy), Stage II (incipient nephropathy), or Stage III (overt nephropathy), wherein a urinary albumin excretion rate "ALB/Cre" of less than 30 mg/g of Cre corresponds to Stage I (pre-nephropathy), a urinary albumin excretion rate from 30 to less than 300 mg/g of Cre corresponds to Stage II (incipient nephropathy), and a urinary albumin excretion rate of 300 mg/g of Cre or more corresponds to Stage III (overt nephropathy).

**5.** A test method according to any one of claims 1 to 4, wherein the detecting of the urinary podocalyxin is carried out by an immunological technique.

**Patentansprüche**

**1.** Ein *in vitro* Testverfahren zur Diagnose von diabetischer Nephropathie umfassend:

Detektieren von im Harn befindlichem, gelösten Podocalyxin, wobei der Podocalyxinwert im Harn mit einer Menge oder Konzentration von Harn-Kreatinin korrigiert wird, in dem die Harn-Podocalyxin-Menge oder - Konzentration durch die Menge bzw. Konzentration des Harn-Kreatinins (creatinine - Cre) geteilt wird, und Feststellen, dass ein zu testenden Subjekts, das einen korrigierten Harn-Podocalyxin Wert hat, der höher als ein Referenzwert ist, diabetische Nephropathie hat, wobei der Referenzwert ein Harn-Podocalyxin-Wert in ein gesunden Subjektes ist.

**2.** Testverfahren nach Anspruch 1, wobei ein frühes Stadium der diabetische Nephropathie detektiert wird, wobei besagtes frühe Stadium durch eine Harn-Albumin(ALB)-Exkretions-Rate "ALB/Cre" von weniger als 30 mg/g Kreatinine charakterisiert ist.

**3.** Testverfahren nach Anspruch 1 oder 2, wobei der Referenzwert einen oberen Grenzwert eines 95% Konfidenzintervalls eines Harn-Podocalyxin-Wertes in ein gesunden Subjekts umfasst.

**4.** Ein Testverfahren für diabetische Nephropathie nach Anspruch 1 oder 2, des Weiteren umfassend das Feststellen des Fortschreitens der diabetische Nephropathie und/oder Klassifizierung der diabetische Nephropathie in Stadium I (vor-Nephropathie), Stadium II (beginnende Nephropathie), oder Stadium III (offenkundige Nephropathie), wobei eine Harn-Albumin(ALB)-Exkretions-Rate "ALB/Cre" von weniger als 30mg/g Cre mit Stadium I (vor-Nephropathie) korrespondiert, eine Harn-Albumin(ALB)-Exkretions-Rate von 30 bis weniger als 300 mg/g Cre mit Stadium II (beginnende Nephropathie) korrespondiert, und eine Harn-Albumin(ALB)-Exkretions-Rate von 300 mg/g Cre oder mehr mit Stadium III (offenkundige Nephropathie) korrespondiert.

**5.** Testverfahren nach einem der Ansprüche 1 bis 4, wobei die Detektion von Harn-Podocalyxin mittels immunologischer Techniken durchgeführt wird.

**Revendications**

**1.** Méthode de test in vitro pour le diagnostic d'une néphropathie diabétique, comprenant:

la détection de podocalyxine urinaire solubilisée, dans laquelle la valeur de podocalyxine urinaire est corrigée avec une quantité ou une concentration de créatinine urinaire par division de la quantité ou la concentration de podocalyxine urinaire par la quantité ou la concentration de créatinine urinaire (Cre), respectivement; et l'évaluation d'un sujet à tester qui a une valeur de podocalyxine urinaire corrigée plus élevée qu'une valeur de référence pour avoir une néphropathie diabétique, tandis que la valeur de référence est une valeur de podocalyxine urinaire chez un sujet en bonne santé.

**2.** Méthode de test selon la revendication 1, dans laquelle un stade précoce de la néphropathie diabétique est détecté, tandis que ledit stade précoce est **caractérisé par** un taux d'excrétion d'albumine urinaire (ALB) "ALB/Cre" inférieur à 30 mg/g de créatinine.

**3.** Méthode de test selon la revendication 1 ou 2, dans laquelle la valeur de référence comprend une valeur limite supérieure d'un intervalle de confiance de 95% d'une valeur de podocalyxine urinaire chez un sujet en bonne santé.

**4.** Méthode de test pour néphropathie diabétique selon la revendication 1 ou 2, comprenant en outre l'évaluation de la progression de la néphropathie diabétique et/ou la classification de la néphropathie diabétique dans le Stade I (pré-néphropathie), le Stade II (néphropathie débutante), ou le Stade III (néphropathie patente), tandis qu'un taux d'excrétion d'albumine urinaire "ALB/Cre" de moins de 30 mg/g de Cre correspond au Stade I (pré-néphropathie), un taux d'excrétion d'albumine urinaire allant de 30 à moins de 300 mg/g de Cre correspond au Stade II (néphropathie débutante), et un taux d'excrétion d'albumine urinaire de 300 mg/g de Cre ou plus correspond au Stade III (néphropathie patente).

5. Méthode de test selon l'une quelconque des revendications 1 à 4, dans laquelle la détection de la podocalyxine urinaire est mise en oeuvre par une technique immunologique.

[Figure 1]

[Figure 2]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007102787 A1
- JP 2008216137 A

- WO 2002037099 A1

**Non-patent literature cited in the description**

- **HARA et al.** *Nephron,* 1995, vol. 69, 397-403
- **NAKAMURA et al.** *Metabolism,* 2001, vol. 50 (10), 1193-1196
- **HARA et al.** *Kidney and Dialysis,* 2008, vol. 65 (4), 488-491

- **NAKAMURA et al.** *Nephrol. Dial. Transplant.,* 2000, vol. 15 (9), 1379-1383
- **SEKINE et al.** *Ther. Res.,* 2008, vol. 29 (11), 1900-1904